# EUROPEAN PATENT SPECIFICATION

(11) **EP 1 866 007 B1**
(45) Date of publication and mention of the grant of the patent: **16.11.2011**
(21) Application number: 06739863.6
(22) Date of filing: 28.03.2006
(51) Int. Cl.: A61L 31/14, A61L 31/16, A61K 9/50, A61F 2/06, B01J 13/00, B05D 3/00

(54) **SYSTEM AND METHOD FOR LOADING A BENEFICIAL AGENT INTO A MEDICAL DEVICE**
SYSTEM UND VERFAHREN ZUM LADEN EINES VORTEILHAFTEN MITTELS IN EIN MEDIZINPRODUKT
SYSTEME ET PROCEDE DE CHARGEMENT D'UN AGENT BENEFIQUE DANS UN DISPOSITIF MEDICAL

(30) Priority: 31.03.2005 US 667564 P
(43) Date of publication of application: 19.12.2007
(73) Proprietor: Innovational Holdings, LLC, New Brunswick, NJ 08933 (US)
(72) Inventor: DIAZ, Stephen Hunter, Palo Alto, California 94301 (US); PARKER, Theodore L., Danville, California 94506 (US)
(74) Representative: Fisher, Adrian John
(86) International application number: PCT/US2006/011346
(87) International publication number: WO 2006/105126

(56) References cited:
- DE-A1- 10 200 387
- US-A1- 2004 238 978
- US-A1- 2005 055 078

## Description

### Field of the Invention

The invention relates to a method and apparatus for loading a beneficial agent, such as a drug into a medical device, such as a stent.

### Description of the Related Art

Implantable medical devices are often used for delivery of a beneficial agent, such as a drug, to an organ or tissue in the body at a controlled delivery rate over an extended period of time. These devices may deliver agents to a wide variety of bodily systems to provide a wide variety of treatments.

One of the many implantable medical devices which have been used for local delivery of beneficial agents is the coronary stent. Coronary stents are typically introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Known stent designs include monofilament wire coil stents (U.S. Pat. No. 4,969,458); welded metal cages (U.S. Pat. Nos. 4,733,665 and 4,776,337); and, most prominently, thin-walled metal cylinders with axial slots formed around the circumference (U.S. Pat. Nos. 4,733,665; 4,739,762; and 4,776,337). Known construction materials for use in stents include polymers, organic fabrics and biocompatible metals, such as stainless steel, gold, silver, tantalum, titanium, and shape memory alloys, such as Nitinol, and biodegradable materials including biodegradable polymers and biodegradable metal alloys.

Of the many problems that may be addressed through stent-based local delivery of beneficial agents, one of the most important is restenosis. Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rates for bare metal stents are still reported in the range of 10% to 25% within six to twelve months after an angioplasty procedure. To treat this condition, additional revascularization procedures are frequently required, thereby increasing trauma and risk to the patient

One of the techniques recently introduced to address the problem of restenosis is the use of surface coatings of various drugs on stents. Surface coatings, however, can provide little actual control over the release kinetics of beneficial agents. These coatings are necessarily very thin, typically 5 to 8 micrometres (microns) deep. The surface area of the stent, by comparison is very large, so that the entire volume of the beneficial agent has a very short diffusion path to discharge into the surrounding tissue.

Increasing the thickness of the surface coating has the beneficial effects of improving drug release kinetics including the ability to control drug release and to allow increased drug loading. However, the increased coating thickness results in increased overall thickness of the stent wall and increased risk of cracking, flaking, or separating from the stent.

In addition, it is not currently possible to deliver many drugs with a surface coating due to sensitivity of the drugs to water, other compounds, or conditions in the body which degrade the drugs. Lack of drug capacity and lack of control over delivery also limit the usefulness of surface coatings for many drugs.

DE 102 00 387 A1 describes a stent having pores. This stent is loaded with a drug by filling the pores with polymeric particles comprising the drug. One embodiment discloses a particle that is prepared to fit in a pore so that knowing the exact quantity of pores, the precise amount of drug loaded in the stent is known. This particle is bonded locally to the pore by mechanical or electrostatic manner.

U.S. Patent Publication 2004/0073294 describes systems and methods for loading a beneficial agent into holes in a medical device, such as a stent. This process uses a computer guided micro dispenser to load droplets of liquid solution into the holes of the stent. The stents are mounted on a rubber coated mandrel blocking the bottoms of the holes. A machine, using machine vision, maps the exact locations of each of the target holes and then moves each hole under the dispenser that then shoots liquid into the holes. The filled stent is dried in an oven, and then a next deposit is applied. Subsequent deposits of polymer and polymer/drug are applied to achieve the desired release properties.

This process has some advantages. It is a non-contact process, so there is little drag of material from hole to hole and no back contamination. It is very fast, filling at least 10 holes per second. The dispenser can be turned on and off very quickly, so complex patterns of filling can be supported. It has proven results of accuracy and consistency.

The liquid droplet method also has some limitations. The piezoelectric dispenser generally requires solutions with low viscosities. Therefore, the solids content should remain low, often less than 5%. The low solids content can result in the need for many deposits to build up a sufficient amount of beneficial agent. In addition, the solid should be very soluble in the solvent. This may require the use of solvents that have undesirable properties. Finally, the oven drying step is too hot for some drugs or proteins.

Accordingly, it would be desirable to provide a system and method for loading a beneficial agent into an expandable medical device, such as a stent, which can deliver compositions with higher solids content and/or can operate with limited drying time or low drying temperature.

### Summary of the Invention

The present invention relates to a system and method for loading a beneficial agent in a medical device wherein the beneficial agent is in the form of particles.

In accordance with one aspect of the invention, a method for loading a medical device with a beneficial agent comprises the steps of providing a medical device with a plurality of holes, delivering a plurality of particles of drug into the plurality of holes in a dry form, and delivering a liquefying substance into the plurality of holes. The liquefying substance liquefies at least a portion of the particles and adheres the drug in the holes.

In accordance with another aspect of the invention, a method for loading a medical device with a beneficial agent comprises the steps of providing a medical device with a plurality of holes, forming a plurality of particles comprising a beneficial agent, delivering the plurality of particles into the plurality of holes, and securing the particles in the holes. The particles are sized to fit as a plug with a single particle in each of the plurality of holes.

In accordance with a further aspect of the invention, a system for loading a medical device with a beneficial agent is comprised of a particle delivery system for delivery of particles of drug into a plurality of holes in a medical device and a solvent delivery system for delivery of a liquid solvent into the plurality of holes in the medical device.

### Brief Description of the Drawings

The invention will now be described in greater detail with reference to the preferred embodiments illustrated in the accompanying drawings, in which like elements bear like reference numerals.
FIGS. 1A-1C are side cross sectional views of a hole in a medical device being loaded by a first method of the present invention.
FIGS. 2A and 2B are side cross sectional views of a hole in a medical device being loaded by a second method of the present invention.
FIG. 3 is a side cross sectional view of a hole in a medical device being loaded by a third method of the present invention.

### Detailed Description of the Invention

The present invention relates to a method and apparatus for loading a beneficial agent into a medical device. More particularly, the invention relates to a method and apparatus for loading a beneficial agent in a stent.

First, the following terms, as used herein, shall have the following meanings:

The term "beneficial agent" as used herein is intended to have its broadest possible interpretation and is used to include any therapeutic agent or drug, as well as inactive agents such as barrier layers, carrier layers, therapeutic layers or protective layers.

The terms "drug" and "therapeutic agent" are used interchangeably to refer to any therapeutically active substance that is delivered to a living being to produce a desired, usually beneficial, effect. The present invention is particularly well suited for the delivery of antineoplastic, angiogenic factors, immuno-suppressants, anti-inflammatories and antiproliferatives (anti-restenosis agents) such as paclitaxel and Rapamycin for example, and antithrombins such as heparin, for example.

The term "matrix" or "biocompatible matrix" are used interchangeably to refer to a medium or material that, upon implantation in a subject, does not elicit a detrimental response sufficient to result in the rejection of the matrix. The matrix typically does not provide any therapeutic responses itself, though the matrix may contain or surround a therapeutic agent, a therapeutic agent, an activating agent or a deactivating agent, as defined herein. A matrix is also a medium that may simply provide support" structural integrity or structural barriers. The matrix may be polymeric, non-polymeric, hydrophobic, hydrophilic, lipophilic, amphiphilic, and the like.

The term "bioresorbable" refers to a matrix, as defined herein, that can be broken down by either chemical or physical process, upon interaction with a physiological environment. The bioresorbable matrix is broken into components that are metabolizable or excretable, over a period of time from minutes to years, preferably less than one year, while maintaining any requisite structural integrity in that same time period.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{w} greater than 3000 and preferably greater than 10,000 and a M_{w} that is less than 10 million, preferably less than a million and more preferably less than 200,000.

The term "holes" refers to holes of any shape and includes both through-holes and recesses.

### Implantable Medical Devices with Holes

U.S. Patent No. 6,241,762 illustrates a medical device in the form of a stent designed with large, non-deforming struts, which can contain holes without compromising the mechanical properties of the struts, or the device as a whole. The non-deforming struts can be achieved by the use of ductile hinges which are described in detail in U.S. Patent No. 6,241,762. The holes serve as large, protected reservoirs for delivering various beneficial agents to the device implantation site.

The holes can be circular, oval, rectangular, polygonal, D-shaped, or other shaped and can extend through the thickness of the medical device. The volume of beneficial agent that can be delivered using holes is 3 to 10 times greater than the volume of a 5 micron coating covering a stent with the same stent/vessel wall coverage ratio. This much larger beneficial agent capacity provides several advantages. The larger capacity can be used to deliver multi-drug combinations, each with independent release profiles, for improved efficacy. Also, larger capacity can be used to provide larger quantities of less aggressive drugs to achieve clinical efficacy without the undesirable side-effects of more potent drugs.

According to one example, the total depth of the holes is 100 to 140 micrometres (microns)(0.0039 to 0.0055 inches), typically 125 micrometres (microns) (0.0049 inches) for stainless steel. For stronger alloys, such as commercially available cobalt chromium alloys, the stent may be somewhat thinner. For example, the total depth of the holes is 60 to 100 micrometres (microns)(0.0026 to 0.0039 inches) for cobalt chromium alloys. According to one preferred embodiment of the present invention, each of the holes have an area of at least 3226 squaremicrometres (5 x 10⁻⁶ square inches), and preferably at least 6452 square micrometres (10 x 10⁻⁶ square inches). A square hole having a width of about 127 micrometres (0.005 inches) will have an hole area of about 16130 square micrometres (25 x 10 square inches).

### Uses for Implantable Medical Devices

Although the present invention has been described with reference to a medical device in the form of a stent, the medical devices of the present invention can also be medical devices of other shapes useful for site-specific and time-release delivery of drugs to the body including the heart and other organs and tissues. The drugs may be delivered to the vasculature including the coronary and peripheral vessels for a variety of therapies, and to other lumens in the body. The drugs may increase lumen diameter, create occlusions, or deliver the drug for other reasons. The medical devices can take a variety of shapes including cylinders, spheres, coils, filament, mesh, and other shapes.

Medical devices and stents, as described herein, are useful for the prevention of amelioration of restenosis, particularly after percutaneous transluminal coronary angioplasty and intraluminal stent placement. In addition to the timed or sustained release of anti-restenosis agents, other agents such as anti-inflammatory agents may be incorporated into the microstructures incorporated in the phuality of holes within the device. This allows for site-specific treatment or prevention any complications routinely associated with stent placements that are known to occur at very specific times after the placement occurs.

### Systems and Methods for Loading a Beneficial Agent into a Medical Device

The beneficial agent is applied into the holes in a medical device in a dry particulate form and is adhered in the hole in a manner that allows release of the drug in a controlled manner.

According to a first embodiment, a machine very similar to the dropwise filling machine described in U.S. Patent Publication 2004/0073294, is used to deliver a slow drying solvent into the holes in a dropwise manner. Alternatively, the slow drying solvent can be delivered into the holes in other manners. The hole should only be partially filled with solvent, for example, the solvent can fill 10 % to 80% of the hole.

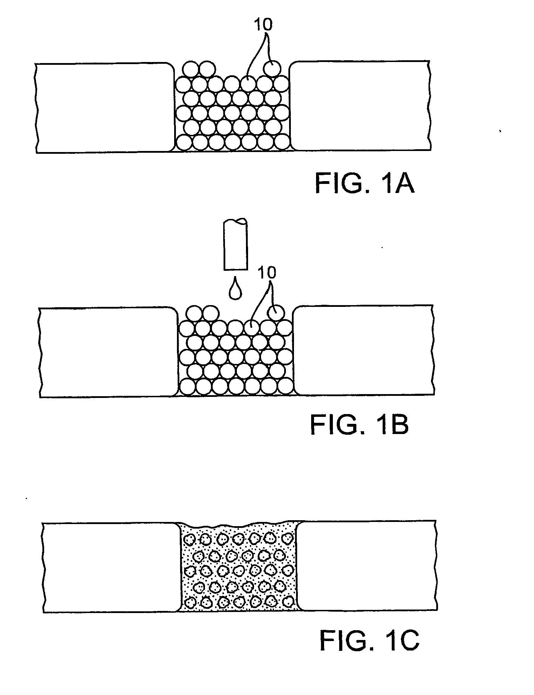

The drug material would be formed into particles 10 and placed in the holes as shown in FIG. 1A. The solvent would be added as shown in FIG. 1B to partially liquefy and adhere the drug into the holes. After application of the solvent, the particles are adhered together in a substantially uniform drug containing matrix, as shown in FIG. 1C. The particles may include drug alone or drug in combination with other materials including a matrix.

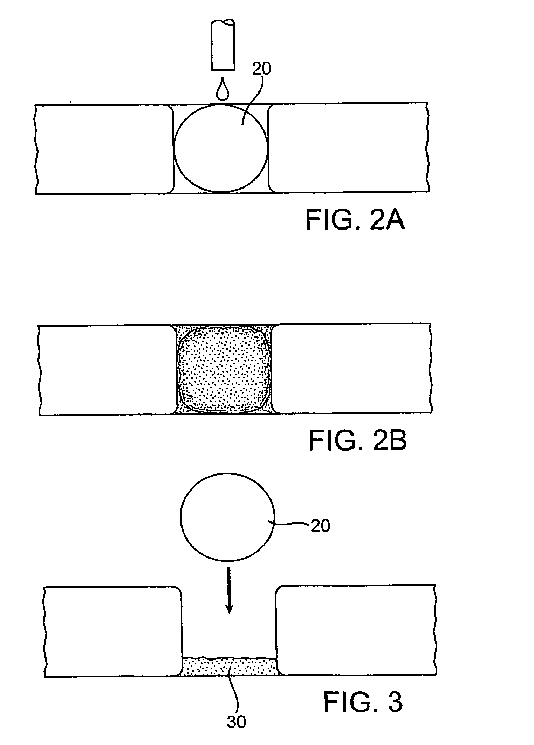

In one example shown in FIGS. 2A and 2B the particles are spheres 20 of about 127 micrometers (0.005 inches) in diameter, so that one sphere will fit in each hole. One example of the formation of spheres which fit with a single sphere in each hole is shown in U.S. Publication No. 2003/0082680. Upon application of the solvent the sphere is adhered within the hole as shown in FIG. 2B.

Alternately, the spheres or particles can be sized smaller so that a plurality of spheres or particles fit in each hole. The particles can be dropped, shot, or sprayed out of a tube that is positioned over the hole. This can be performed with a computer controlled jetting device, such as a piezoelectric microjet. In another embodiment of FIG. 3 the solvent 30 is first applied to the hole, such as with a dropwise filler. The sphere 20 then drops into the solvent and sticks in the hole. The solvent then causes the sphere to soften, expand, and become bonded or adhered to the walls of the hole.

The release kinetics of the microstructure created within the holes can be modified by using multi-layer spheres with the layers having different compositions to control the release. For example, a sphere with a central drug/polymer core can be surrounded by a polymer only layer with can form a barrier layer to control delivery of the drug.

As an alternative to depositing the particles in the holes by a controlled jetting process, the holes can be loaded with particles by dipping. For example, a mandrel having one or more stents with solvent filled holes can be immersed in an ocean of particles or spheres, such as in a fluidized bed. The particles that contacted the solvent at the bottom of the holes will stick there, and the others will fall off.

Yet another method of applying the particles is to use an electrostatic spraying method to apply the particles into the holes. The mandrel can be charged and the charged particles can be sprayed onto the stents. The sprayed spheres will stick all over the stents, but only the spheres falling into the holes will be bonded in place on the stent by the liquefying agent or solvent within the holes.

In an alternative embodiment, the process is reversed and the spheres are applied first. When the holes are slightly square or tapered (as they naturally are due to the laser cutting process), and if the spheres are just the right size, then they can be placed or wedged in the holes, but nowhere else on the stent. If the right combination of size distributions is achieved, and if the spheres are shot at the stent with some velocity, every hole will have a sphere stuck in it, perhaps with the aid of a static charge. After blowing or brushing off the excess spheres, the entire mandrel can be sprayed with solvent to soften the spheres lodged in the holes to force them to stick in place. Alternately, the solvent can be shot dropwise into the individual holes after filling them with spheres, as before.

Other particles including a simple powder or chopped fiber can be used as an alternative to the spheres. The concept with powder or other particles remains the same as with the spheres. Selective adhesion of the drug or drug/polymer matrix in the hole combined with a solvent or weak solution applied before or after application of the particles allows the drug or drug/polymer material to be permanently affixed in the holes.

In one embodiment, the particles and liquefying agents delivered into the holes can be loaded sequentially in layers with different compositions or concentrations in the layers. Different layers can be comprised of different therapeutic agents altogether, creating the ability to release different therapeutic agents at different points in time. The layers of beneficial agent provide the ability to tailor a drug delivery profile to different applications. This allows the medical device according to the present invention to be used for delivery of different beneficial agents to a wide variety of locations in the body.

A protective layer in the form of a cap layer can be provided at a tissue contacting surface of the stent. The cap layer can block or retard biodegradation of subsequent layers and/or block or retard diffusion of the beneficial agent in that direction for a period of time which allows the delivery of the medical device to a desired location in the body.

A barrier or base layer can also be used on the luminal (or mural) surface of the stent to achieve directional delivery of the therapeutic agent. The barrier or base layer can prevent the therapeutic agent from passing into the lumen and being carried away in the blood stream.

Other therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, AZ blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, vasodilators, and other miscellaneous compounds.

Antiproliferatives include, without limitation, paclitaxel, actinomycin D, rapamycin, everolimus, ABT-578, tacrolimus, cyclosporin, and pimecrolimus.

Antithrombins include, without limitation, heparin, aspirin, sulfinpyrazone, ticlopidine, ABCIXIMAB, eptifibatide, tirofiban HCL, coumarines, plasminogen, α₂-antiplasmin, streptokinase, urokinase, bivalirudin, tissue plasminogen activator (t-PA), hirudins, hirulogs, argatroban, hydroxychloroquin, BL-3459, pyridinolcarbamate, Angiomax, and dipyridamole.

Immunosuppressants include, without limitation, cyclosporine, rapamycin and tacrolimus (FK-506), ABT-578, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, pimecrolimus, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, spirapril, and 2B3 ACE inhibitors.

Cell sensitizers to insulin include, without limitation, glitazones, P PAR agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, paclitaxel derivatives (e.g., docetaxel), rapamycin, rapamycin derivatives, everolimus, tacrolimus, ABT-578, and pimecrolimus.

PPAR gamma agonists include, without limitation, farglitizar, rosiglitazone, muraglitazar, pioglitazone, troglitazone, and balaglitazone.

Miscellaneous compounds include, without limitation, Adiponectin.

Agents may also be delivered using a gene therapy-based approach in combination with an expandable medical device. Gene therapy refers to the delivery of exogenous genes to a cell or tissue, thereby causing target cells to express the exogenous gene product. Genes are typically delivered by either mechanical or vector-mediated methods.

Some of the agents described herein may be combined with additives which preserve their activity. For example additives including surfactants, antacids, antioxidants, and detergents may be used to minimize denaturation and aggregation of a protein drug. Anionic, cationic, or nonionic detergents may be used. Examples of nonionic additives include but are not limited to sugars including sorbitol, sucrose, trehalose; dextrans including dextran, carboxy methyl (CM) dextran, diethylamino methyl (DEAE) dextran; sugar derivatives including D-glucosaminic acid, and D-glucose diethyl mercaptal; synthetic polyethers including polyethylene glycol (PEF and PEO) and polyvinyl pyrrolidone (PVP); carboxylic acids including D-lactic acid, glycolic acid, and propionic acid; detergents with affinity for hydrophobic interfaces including n-dodecyl-β-maltoside, n-octyl-β-D-glucoside, PEO-fatty acid esters (e.g. stearate (myrj 59) or oleate), PEO-sorbitan-fatty acid esters (e.g. Tween 80, PEO-20 sorbitan monooleate), sorbitan-fatty acid esters (e.g. SPAN 60, sorbitan monostearate), PEO-glyceryl-fatty acid esters; glyceryl fatty acid esters (e.g. glyceryl monostearate), PEO-hydrocarbon-ethers (e.g. PEO-10 oleyl ether, triton X-100; and Lubrol. Examples of ionic detergents include but are not limited to fatty acid salts including calcium stearate, magnesium stearate, and zinc stearate; phospholipids including lecithin and phosphatidyl choline; CM-PEG; cholic acid; sodium dodecyl sulfate (SDS); docusate (AOT); and taumocholic acid.

## Claims

1. A method for loading a medical device with a beneficial agent, the method comprising: providing a medical device with a plurality of holes; delivering a plurality of particles of drug into the plurality of holes in a dry form; and delivering a liquefying substance into the plurality of holes, wherein the liquefying substance liquefies at least a portion of the particles and adheres the drug in the holes.

2. The method of Claim 1 , wherein the plurality of particles of drug comprise spheres of drug.

3. The method of Claim 2, wherein the spheres are sized to fit with a single sphere per hole.

4. The method of Claim 2, wherein the spheres comprise a drug core and a polymer shell.

5. The method of Claim 2, wherein the spheres comprise a mixture of drug and carrier.

6. The method of Claim 2, wherein the spheres have a size of 2.5 to 1270 micrometers (0.0001 to 0.05 inches).

7. The method of Claim 1 , wherein the plurality of particles include a carrier.

8. The method of Claim 7, wherein the carrier is a polymer.

9. The method of Claim 8, wherein the plurality of particles of drug and polymer comprise a powder.

10. The method of Claim 1, wherein the liquefying substance is a solvent.

11. The method of Claim 1, wherein the liquefying substance is delivered into the plurality of holes before the particles are delivered to the holes.

12. The method of Claim 1, wherein the liquefying substance is delivered into the plurality of holes after the particles are delivered to the holes.

13. The method of Claim 1, wherein the particles are delivered into the holes in a plurality of layers.

14. The method of Claim 1, wherein the particles are delivered into the holes by an electrostatic process.

15. The method of Claim 1, wherein the liquefying substance is delivered into the plurality of holes in a dropwise manner.

16. The method of Claim 14, wherein the liquefying substance is delivered into the plurality of holes by a computer controlled jetting process.

17. The method of Claim 11, wherein the particles are delivered into the plurality of holes by immersion of the stent into the particles.

18. A system for loading a medical device with a beneficial agent, the system comprising: a particle delivery system for delivery of particles of drug into a plurality of holes in a medical device; a solvent delivery system for delivery of a liquid solvent into the plurality of holes in the medical device.

## Patentansprüche

1. Verfahren zum Beladen einer medizinischen Vorrichtung mit einem Wirkstoff, wobei das Verfahren umfasst: Bereitstellen einer medizinischen Vorrichtung mit einer Vielzahl von Löchern; Zuführen einer Vielzahl von Arzneimittelteilchen in trockener Form in die Vielzahl von Löchern; und Zuführen eines verflüssigenden Stoffs in die Vielzahl von Löchern, wodurch der verflüssigende Stoff wenigstens einen Teil der Teilchen verflüssigt und das Arzneimittel in den Löchern anhaftet.

2. Verfahren gemäß Anspruch 1, wobei die Vielzahl von Arzneimittelteilchen Arzneimittelkugeln umfasst.

3. Verfahren gemäß Anspruch 2, wobei die Kugeln so groß sind, dass sie als einzelne Kugel in ein Loch passen.

4. Verfahren gemäß Anspruch 2, wobei die Kugeln einen Arzneimittelkern und eine Polymerhülle umfassen.

5. Verfahren gemäß Anspruch 2, wobei die Kugeln ein Gemisch von Arzneimittel und Träger umfassen.

6. Verfahren gemäß Anspruch 2, wobei die Kugeln eine Größe von 2,5 bis 1270 Mikrometer (0,0001 bis 0,05 Inch) aufweisen.

7. Verfahren gemäß Anspruch 1, wobei die Vielzahl von Teilchen einen Träger umfasst.

8. Verfahren gemäß Anspruch 7, wobei der Träger ein Polymer ist.

9. Verfahren gemäß Anspruch 8, wobei die Vielzahl von Arzneimittel-und-Polymer-Teilchen ein Pulver umfasst.

10. Verfahren gemäß Anspruch 1, wobei der verflüssigende Stoff ein Lösungsmittel ist.

11. Verfahren gemäß Anspruch 1, wobei der verflüssigende Stoff der Vielzahl von Löchern zugeführt wird, bevor die Teilchen den Löchern zugeführt werden.

12. Verfahren gemäß Anspruch 1, wobei der verflüssigende Stoff der Vielzahl von Löchern zugeführt wird, nachdem die Teilchen den Löchern zugeführt werden.

13. Verfahren gemäß Anspruch 1, wobei die Teilchen den Löchern in einer Vielzahl von Schichten zugeführt werden.

14. Verfahren gemäß Anspruch 1, wobei die Teilchen den Löchern durch ein elektrostatisches Verfahren zugeführt werden.

15. Verfahren gemäß Anspruch 1, wobei der verflüssigende Stoff der Vielzahl von Löchern tropfenweise zugeführt wird.

16. Verfahren gemäß Anspruch 14, wobei der verflüssigende Stoff der Vielzahl von Löchern durch ein computergesteuertes Jetverfahren zugeführt wird.

17. Verfahren gemäß Anspruch 11, wobei die Teilchen der Vielzahl von Löchern durch Eintauchen des Stents in die Teilchen zugeführt werden.

18. System zum Beladen einer medizinischen Vorrichtung mit einem Wirkstoff, wobei das System umfasst: ein Teilchenzufuhrsystem zum Zuführen von Arzneimittelteilchen in eine Vielzahl von Löchern in einer medizinischen Vorrichtung; ein Lösungsmittelzufuhrsystem zum Zuführen eines flüssigen Lösungsmittels in die Vielzahl von Löchern in der medizinischen Vorrichtung.

## Revendications

1. Procédé pour charger un dispositif médical avec un agent bénéfique, le procédé comprenant : la fourniture d'un dispositif médical avec une pluralité de trous ; la délivrance d'une pluralité de particules de médicament dans la pluralité de trous sous une forme sèche ; et la délivrance d'une substance liquéfiante dans la pluralité de trous, où la substance liquéfiante liquéfie au moins une partie des particules et fait adhérer le médicament dans les trous.

2. Procédé de la revendication 1, dans lequel la pluralité de particules de médicament comprennent des sphères de médicament.

3. Procédé de la revendication 2, dans lequel les sphères sont dimensionnées de manière à s'ajuster avec une seule sphère par trou.

4. Procédé de la revendication 2, dans lequel les sphères comprennent un noyau de médicament et une enveloppe de polymère.

5. Procédé de la revendication 2, dans lequel les sphères comprennent un mélange de médicament et de véhicule.

6. Procédé de la revendication 2, dans lequel les sphères ont une taille de 2,5 à 1270 micromètres (0,0001 à 0,05 pouce).

7. Procédé de la revendication 1, dans lequel la pluralité de particules comprennent un véhicule.

8. Procédé de la revendication 7, dans lequel le véhicule est un polymère.

9. Procédé de la revendication 8, dans lequel la pluralité de particules de médicament et de polymère comprennent une poudre.

10. Procédé de la revendication 1, dans lequel la substance liquéfiante est un solvant.

11. Procédé de la revendication 1, dans lequel la substance liquéfiante est délivrée dans la pluralité de trous avant que les particules soient délivrées dans les trous.

12. Procédé de la revendication 1, dans lequel la substance liquéfiante est délivrée dans la pluralité de trous après que les particules soient délivrées dans les trous.

13. Procédé de la revendication 1, dans lequel les particules sont délivrées dans les trous dans une pluralité de couches.

14. Procédé de la revendication 1, dans lequel les particules sont délivrées dans les trous par un processus électrostatique.

15. Procédé de la revendication 1, dans lequel la substance liquéfiante est délivrée dans la pluralité de trous goutte à goutte.

16. Procédé de la revendication 14, dans lequel la substance liquéfiante est délivrée dans la pluralité de trous par un procédé de jet contrôlé par ordinateur.

17. Procédé de la revendication 11, dans lequel les particules sont délivrées dans la pluralité de trous par immersion de l'endoprothèse dans les particules.

18. Système pour charger un dispositif médical avec un agent bénéfique, le système comprenant : un système de délivrance de particules pour la délivrance de particules de médicament dans une pluralité de trous dans un dispositif médical ; un système de délivrance de solvant pour la délivrance d'un solvant liquide dans la pluralité de trous dans le dispositif médical.
